(19) Europäisches Patentamt
European Patent Office
Office européen des brevets

(11) Numéro de publication: **0 099 799**
**B1**

# (12) FASCICULE DE BREVET EUROPEEN

(45) Date de publication du fascicule du brevet: **12.03.86**

(51) Int. Cl.⁴: **C 07 D 213/66**, A 61 K 31/44

(21) Numéro de dépôt: **83401373.2**

(22) Date de dépôt: **04.07.83**

(54) **Nouveaux sels d'amino- (et imino-) acides alpha, omega-sulfoniques N-substitués, vecteurs cationiques de haute pénétration cellulaire, procédé d'obtention.**

(30) Priorité: **05.07.82 FR 8211768**

(43) Date de publication de la demande: **01.02.84 Bulletin 84/5**

(45) Mention de la délivrance du brevet: **12.03.86 Bulletin 86/11**

(84) Etats contractants désignés: **AT BE CH DE FR GB IT LI LU NL SE**

(56) Documents cités:
**CHEMICAL ABSTRACTS, vol. 83, 1975, page 435, no. 146652z, Columbus, Ohio, USA R. RAUNIO et al.: "Spectral properties of Schiff bases prepared from pyridoxal and various amino compounds"**

(73) Titulaire: **LES LABORATOIRES MERAM Société Anonyme dite:, 4 Bld Malesherbes, F-75008 Paris (FR)**

(72) Inventeur: **Durlach, Jean Pierre, 2, rue Villersexel, F-75007 Paris (FR)**

(74) Mandataire: **Gillard, Marie-Louise et al, Cabinet Beau de Loménie 55, Rue d'Amsterdam, F-75008 Paris (FR)**

EP 0 099 799 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La taurine (acide 2-amino-éthane-sulfonique) et l'homotaurine (acide 3-amino-propane-sulfonique) représentent, en médecine, deux exemples d'amino-acides α, ω-sulfoniques dont l'administration par voie orale se révèle peu efficace.

On a pensé que, en préparant de multiples dérivés de ces composés amphotères à polarité modifiée par la N-substitution, on pourrait obtenir une efficacité plus grande de l'administration par voie orale de ces amino-acides α, ω-sulfoniques.

Le mode de préparation de ces produits ne permettant pas l'obtention des acides sulfoniques, mais des sulfonates, on a constaté, de façon tout à fait inattendue, une propriété singulière propre aux sels alcalins et alcalino-terreux des amino-acides N-substitués par le pyridoxyle, de même que de leurs intermédiaires de synthèse iminiques. Alors que l'on cherchait à modifier les propriétés des amino-acides, on a constaté que ce sont les cations des sels préparés qui se trouvent présenter une propriété inattendue de pénétration exceptionnellement élevée dans l'organisme, propriété particulièrement précieuse lorsqu'elle concerne des cations dont les formes actuellement disponibles en thérapeutique sont d'efficacité médiocre, comme c'est le cas par exemple pour les sels de magnésium.

Or, cette propriété commune aux dérivés N-pyridoxyl-substitués des sels des acides α, ω-amino-(ou imino-)éthane (et propane)-sulfoniques est singulière, car elle ne se retrouve pas chez de nombreux autres dérivés N-substitués différemment. Commune au seul groupe de composés faisant l'objet de l'invention, elle permet d'utiliser ceux-ci comme vecteurs de leurs cations, les différents composés selon l'invention présentant en outre, selon leur structure, notamment saturée ou non, et selon la nature de leurs cations, des propriétés propres à chacun d'eux.

L'invention a donc pour objet les sels des acides N-pyridoxylidène-amino-alcane-sulfoniques de formule:

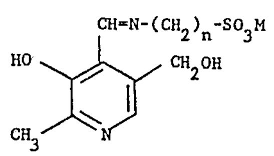

(ci-dessous désignés «composés imino»)

et des acides N-pyridoxyl-amino-alcane-sulfoniques de formule:

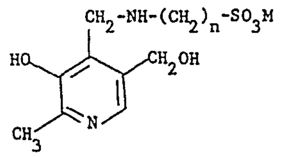

(ci-dessous désignés «composés amino»)

formules dans lesquelles n = 2 ou 3 et M est un métal alcalin (Na, K, Li, Rb, Cs) ou alcalino-terreux (Ca, Mg, Ba, Sr, etc.), et leur application comme médicaments.

A titre d'exemple, on décrira ci-après le mode de préparation des sels de sodium, de potassium, de calcium et de magnésium des dérivés N-pyridoxylidène- et N-pyridoxyl-substitués des acides 2-amino-éthane- et 3-amino-propane-sulfoniques.

*Préparation des composés selon l'invention:*

*A) Pour les sels de métaux alcalins*

Le procédé de préparation consiste, pour obtenir le composé imino ou base de Schiff, à faire réagir le pyridoxal base avec le sel de l'acide α, ω-amino-sulfonique en présence d'une base en milieu alcoolique.

Le dérivé imino est ensuite réduit en dérivé amino correspondant par un borohydrure alcalin.

*Exemple 1*

*Préparation du N-[(méthyl-2 hydroxy-3 hydroxyméthyl-5 pyridyl-4)-méthylène] β-imino-éthane-sulfonate de sodium*

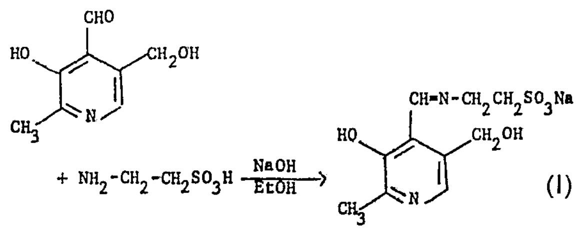


(I)

A 8 g (0,2 mol) de soude, on ajoute sous agitation 20 cm³ d'eau et 25 g (0,2 mol) de taurine puis, une fois la solubilisation obtenue, on ajoute 300 cm³ d'alcool éthylique absolu. On chauffe à 60° C et ajoute d'un coup 33,4 g (0,2 mol) de pyridoxal base finement broyé. On chauffe à 60° C pendant 30 min et laisse descendre la température à 20° C; on essore le précipité jaune, rince avec 200 cm³ d'éthanol absolu, sèche en étuve ventilée à 60° C et obtient 55 g de produit (I), soit 93% de rendement.

*Analyse:*
F. > 250° C.
Soluble dans l'eau.
Insoluble dans l'acétone et l'alcool.

| | C | H | N | S |
|---|---|---|---|---|
| Calculé: | C 40,5 | H 4,38 | N 9,45 | S 10,8% |
| Trouvé: | C 40,7 | H 4,4 | N 9,38 | S 10,8% |

*Exemple 2*

*Préparation du N-[(méthyl-2 hydroxy-3 hydroxyméthyl-5 pyridyl-4)-méthyl]-β-amino-éthane-sulfonate de sodium*

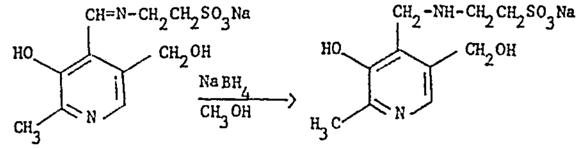


A 150 cm³ de méthanol, on ajoute 29,6 g (0,1 mol) de composé obtenu dans l'exemple 1 broyé finement. Sous agitation et par petites fractions, on additionne 1,8 g (0,05 mol) de borohydrure de sodium en maintenant la température à 20° C. Une fois le borohydrure introduit, on agite

1 h à 20° C, on filtre l'insoluble et concentre le filtrat à sec. On obtient 30 g environ de produit qui sont recristallisés dans 2 volumes de méthanol. Après filtration, on obtient 25 g (84%) de cristaux blancs.

*Analyse:*

F. > 250° C
Soluble dans l'eau.
Peu soluble dans le méthanol.
Insoluble dans l'acétone.

| | | | | |
|---|---|---|---|---|
| Calculé: | C 40,24 | H 5,03 | N 9,39 | S 10,73% |
| Trouvé: | C 41 | H 4,95 | N 9,41 | S 10,8% |

*Exemple 3*

*N-[(méthyl-2 hydroxy-3 hydroxy-méthyl-5 pyridyl-4)-méthylène]β-imino-éthane-sulfonate de potassium*

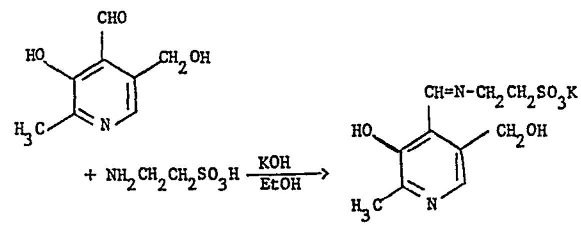


A 5,6 g de potasse, on ajoute sous agitation 10 cm³ d'eau et 12,5 g (0,1 mol) de taurine. Une fois la solubilisation obtenue, on ajoute 150 cm³ d'éthanol absolu, chauffe à 60° C et ajoute en une seule fois 16,7 g (0,1 mol) de pyridoxal base finement broyé. On chauffe à 60° C pendant 30 min puis laisse descendre à 20° C; essore rapidement le précipité jaune, rince avec 100 cm³ d'éthanol absolu et sèche en étuve ventilée à 60° C. On obtient 27,7 g, soit 89% de rendement de produit.

*Analyse:*

F. > 250° C.
Soluble dans l'eau.
Insoluble dans l'acétone.

| | | | | |
|---|---|---|---|---|
| Calculé: | C 38,46 | H 4,16 | N 8,97 | S 10,25% |
| Trouvé: | C 38,1 | H 4,2 | N 10 | S 10,16% |

*Exemple 4*

*N-[(méthyl-2 hydroxy-3 hydroxy-méthyl-5 pyridyl-4)-méthyl]β-amino-éthane-sulfonate de potassium*

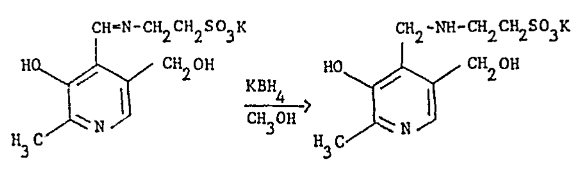


A 150 cm³ de méthanol, on ajoute 32 g (0,1 mol) du composé de l'exemple 3 broyé finement. Sous agitation et par petites fractioins, on additionne 2,7 g (0,05 mol) de borohydrure de potassium en maintenant la température à 20° C. Une fois le borohydrure introduit, on laisse agiter 1 h à 20° C, filtre l'insoluble, concentre le filtrat à sec. On obtient 31 g environ de produit qui sont recristallisés dans 2 volumes de méthanol. Après filtration, on obtient 27 g de cristaux blancs (86% de rendement).

*Analyse:*

F. > 250° C.
Soluble dans l'eau.
Insoluble dans l'acétone.

| | | | | |
|---|---|---|---|---|
| Calculé: | C 38,2 | H 4,77 | N 8,91 | S 10,19% |
| Trouvé: | C 38,7 | H 5,1 | N 9 | S 10,3% |

*Exemple 5*

*N-[(méthyl-2 hydroxy-3 hydroxyméthyl-5 pyridyl-4)-méthylène]γ-imino-propane-sulfonate de sodium*

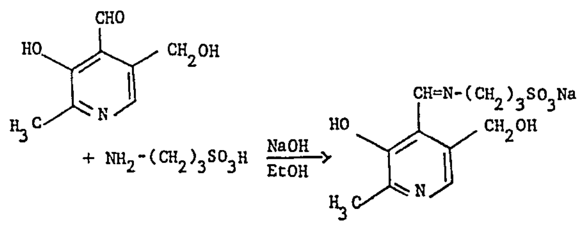


A une solution de 4 g de NaOH (0,1 mol) dans 10 cm³ d'eau, on ajoute 13,9 g (0,1 mol) d'homotaurine. On agite jusqu'à dissolution complète et on ajoute 150 cm³ d'éthanol absolu, chauffe à 60° C pour dissoudre, ajoute en une seule fois 16,7 g (0,1 mol) de pyridoxal base finement broyé. Il y a dissolution puis précipitation. On agite encore 30 min à 60° C, laisse refroidir à 20° C et essore à +5° C. On réempâte dans l'éthanol absolu, essore, sèche à l'étuve sous vide à 60° C.

On obtient 25,5 g de cristaux jaunes. Rendement 82%.

*Analyse:*

Soluble dans l'eau.
Insoluble dans l'acétone et l'alcool éthylique.

| | | | | |
|---|---|---|---|---|
| Calculé: | C 42,55 | H 4,83 | N 9,02 | S 10,31% |
| Trouvé: | C 43,1 | H 4,97 | N 9,1 | S 10,4% |

*Exemple 6*

*N-[(méthyl-2 hydroxy-5 hydroxyméthyl-5 pyridyl-4)-méthyl]γ-amino-propane-sulfonate de sodium*

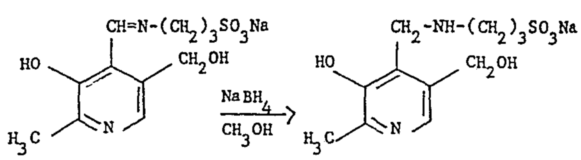


A 150 cm³ de méthanol, on ajoute 31 g (0,1 mol) de composé de l'exemple 5 broyé finement. Sous agitation et par petites fractions, on additionne 1,8 g (0,05 mol) de borohydrure de sodium en maintenant la température à 20° C. Une fois le borohydrure introduit, on agite 1 h à 20° C, filtre l'insoluble, concentre le filtrat à sec. On obtient 31 g environ de produit qui sont recristallisés dans 2 volumes de méthanol. Après filtration, on obtient 28 g (91%) de cristaux blancs.

*Analyse:*

Soluble dans l'eau.
Insoluble dans l'acétone.

| | | | | |
|---|---|---|---|---|
| Calculé: | C 42,2 | H 4,79 | N 8,95 | S 10,23% |
| Trouvé: | C 43,1 | H 5,01 | N 9,1 | S 10,44% |

*Exemple 7*

*N-[(méthyl-2 hydroxy-3 hydroxyméthyl-5 pyridyl-4)-méthylène]γ-imino-propane-sulfonate de potassium*

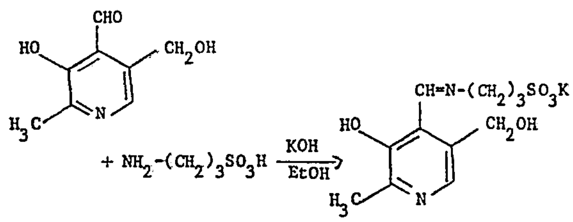


A une solution de 6,16 g (0,11 mol) de potasse dans 10 cm³ d'eau, on ajoute sous agitation 13,9 g (0,1 mol) d'homotaurine puis, quand la dissolution est totale, 150 cm³ d'éthanol absolu. On chauffe à 60° C et ajoute en une seule fois 16,7 g (0,1 mol) de pyridoxal base finement broyé. Il y a dissolution. On maintient à 60° C pendant 30 min, amorce la cristallisation en grattant ou en ensemençant et essore à 5° C. On réempâte dans l'éthanol absolu, sèche à l'étuve sous vide à 60° C.

On obtient 27,3 g (83,7%) de cristaux jaunes hygroscopiques.

*Analyse:*

Soluble dans l'eau.
Insoluble dans l'acétone.

| | | | | |
|---|---|---|---|---|
| Calculé: | C 40,47 | H 4,59 | N 8,58 | S 9,81% |
| Trouvé: | C 39,98 | H 4,38 | N 8,52 | S 9,91% |

*Exemple 8*

*N-[(méthyl-2 hydroxy-3 hydroxyméthyl-5 pyridyl-4)-méthyl]γ-amino-propane-sulfonate de potassium*

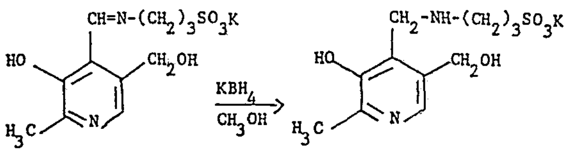


A 150 cm³ de méthanol, on ajoute 32,6 g (0,1 mol) de composé de l'exemple 7 broyé finement. Sous agitation et par petites fractions, on ajoute 2,7 g (0,05 mol) de borohydrure de potassium en maintenant la température à 20° C. Une fois le borohydrure introduit, on agite 1 h à 20° C, filtre l'insoluble et concentre à sec. On obtient 33 g environ de produit qui sont recristallisés dans 2 volumes de méthanol. Après filtration, on obtient 29,5 g (90%) de cristaux blancs.

*Analyse:*

Soluble dans l'eau.
Insoluble dans l'acétone.

| | | | | |
|---|---|---|---|---|
| Calculé: | C 40,22 | H 5,18 | N 8,53 | S 9,75% |
| Trouvé: | C 39,8 | H 4,89 | N 8,32 | S 9,36% |

On peut, par le même procédé que celui décrit pour les sels de sodium et de potassium, préparer les sels d'autres métaux alcalins tels que Li, Rb, Cs.

*Exemple 9*

*N-[(méthyl-2 hydroxy-3 hydroxyméthyl-5 pyridyl-4)-méthylène]β-imino-éthane-sulfonate de lithium*

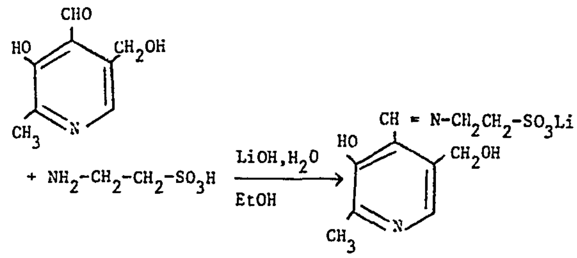


A 4,2 g de lithine (0,1 mol), on ajoute sous agitation 10 cm³ d'eau et 12,5 g (0,1 mol) de taurine. On chauffe à 60° C pour dissoudre et on ajoute 150 cm³ d'éthanol absolu. On maintient à 60° C et on ajoute 16,7 g de pyridoxal (0,1 mol) finement broyé. On chauffe à 60° C pendant 30 min et on laisse descendre à 20° C, puis on refroidit à 0° C. On essore rapidement le précipité jaune, on rince avec 100 cm³ d'éthanol. On sèche à l'étuve ventilée à 60° C.

On obtient 23,8 g, soit un rendement de 85%.

*Analyse:*

Point de fusion > 250° C.
Soluble dans l'eau.
Insoluble dans l'acétone.

| | | | | |
|---|---|---|---|---|
| Calculé: | C 42,8 | H 4,64 | N 10 | O 28,5 |
| | S 11,42 | Li 2,5% | | |
| Trouvé: | C 42,04 | H 4,52 | N 9,9 | O 28,42 |
| | S 11,43 | Li 2,48% | | |

Selon le même mode opératoire, on a préparé le N-[(méthyl-2 hydroxy-3 hydroxyméthyl-5 pyridyl-4)-méthylène]γ-imino-propane-sulfonate de lithium, dont le point de fusion est supérieur à 250° C; il est soluble dans l'eau et insoluble dans l'acétone.

*Exemple 10*

*N-[(méthyl-2 hydroxy-3 hydroxyméthyl-5 pyridyl-4)-méthyl]β-amino-éthane-sulfonate de lithium*

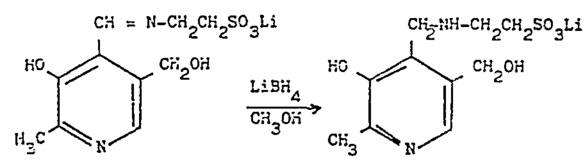


A 150 cm³ de méthanol, on ajoute 28 g (0,1 mol) du composé obtenu à l'exemple 9 finement broyé. Sous agitation et par petites fractions, on additionne 1,1 g (0,05 mol) de borohydrure de lithium en maintenant la température à 20° C. On filtre l'insoluble, on concentre le filtrat à sec.

On obtient 28 g environ qui sont recristallisés dans 2 volumes de méthanol. Après filtration, on obtient 27 g (96%) de cristaux blancs.

*Analyse:*

Soluble dans l'eau.
Insoluble dans l'acétone.

Calculé: C 42,5 H 5,35 N 9,92 O 28,36 S 11,34 Li 2,48%

Trouvé: C 42,38 H 5,29 N 10 O 28,62 S 11,38 Li 2,46%

Selon le même mode opératoire, on a également préparé le N-[(méthyl-2 hydroxy-3 hydroxyméthyl-5 pyridyl-4)-méthyl]γ-amino-propane-sulfonate de lithium, qui est soluble dans l'eau et insoluble dans l'acétone.

*B) Pour préparer les sels de métaux alcalino-terreux*

On forme d'abord le sel alcalino-terreux du composé imino par échange entre un sel alcalin du composé imino préparé selon A, par exemple le sel de sodium et un sel alcalino-terreux, par exemple un chlorure alcalino-terreux, puis on réduit le composé imino en composé amino correspondant.

*Exemple 11*

*N-[(méthyl-2 hydroxy-3 hydroxyméthyl-5 pyridyl-4)-méthylène]β-imino-éthane-sulfonate de calcium*

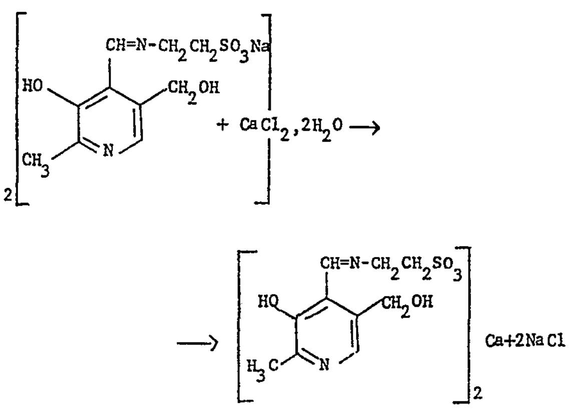


26 de composé de l'exemple 1 (0,0878 mol) sont dissous dans 50 cm³ d'eau. On ajoute d'un coup une solution de 6,5 g (0,0439 mol) de chlorure de calcium déshydraté dans 30 cm³ d'eau. On agite 30 min et on filtre sur papier. Le filtrat est concentré à sec, on obtient un solide rouge qui est dissous dans 100 cm³ de méthanol. On filtre le chlorure de sodium et le filtrat est concentré à sec, puis séché à l'étuve sous vide.

On obtient 25 g (97%) de produit orangé.

*Analyse:*

F. > 250° C
Cristaux amorphes orangés.
Soluble dans l'eau.
Insoluble dans l'acétone.

Calculé: C 40,95 H 4,43 N 9,55 S 10,92 O 27,3 Ca 6,82%

Trouvé: C 41,2 H 4,5 N 9,6 S 11 Ca 6,78%

*Exemple 12*

*N-[(méthyl-2 hydroxy-3 hydroxyméthyl-5 pyridyl-4)-méthylène]γ-imino-propane-sulfonate de calcium*

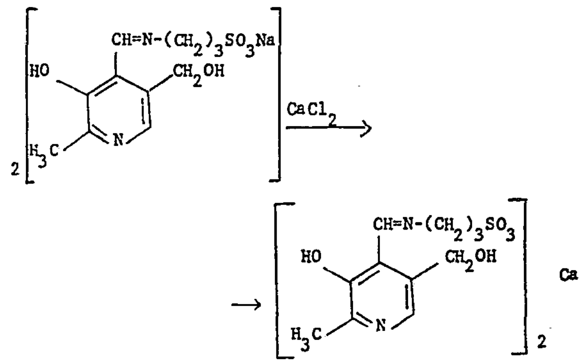


A une solution de 15,5 g (0,05 mol) du sel de sodium de l'exemple 5 dans 30 cm³ d'eau, on ajoute une solution de 3,7 g (0,025 mol) de chlorure de calcium déshydraté dans 20 cm³ d'eau.

On agite 15 min et on filtre un léger trouble. On concentre à sec ensuite et onreprend avec 100 cm³ de méthanol bouillant, filtre le chlorure de sodium et concentre le filtrat à sec. On obtient 15,3 g de sel orange. Rendement 100%.

*Analyse:*

Soluble dans l'eau.
Insoluble dans l'acétone.
Soluble dans le méthanol.
$C_{22}H_{30}N_4S_2O_{10}Ca = 614,32$

Calculé: C 42,97 H 4,88 N 9,11 S 10,41 Ca 6,51%

Trouvé: C 43,01 H 4,91 N9,08 S 10,38 Ca 6,49%

Teneur en eau résiduelle = 3,7%

*Exemple 13*

*N-[(méthyl-2 hydroxy-3 hydroxyméthyl-5 pyridyl-4)-méthylène]γ-imino-éthane-sulfonate de magnésium*

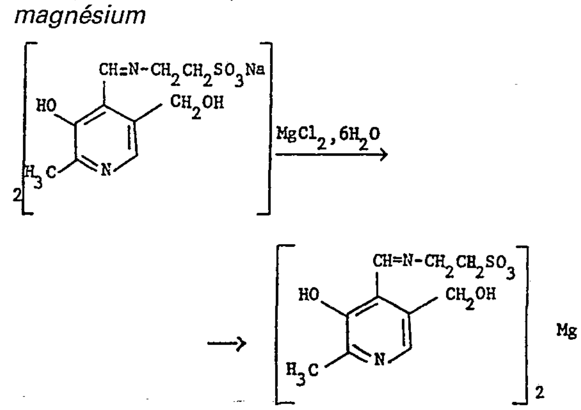


29,6 g du sel de sodium (0,1 mol) de l'exemple 1 sont mis en suspension dans 180 cm³ de méthanol. On ajoute alors une solution de 10,2 g (0,05 mol) de chlorure de magnésium hydraté dans 20 cm³ de méthanol. Il y a dissolution presque complète et précipitation du chlorure de sodium.

On agite pendant 30 min et on filtre. Le filtrat est concentré à sec et déshydraté sous vide. Le solide est repris avec 100 $cm^3$ de $CH_3OH$ bouillant et essoré à chaud.

On sèche à l'étuve sous vide à 80° C.

On obtient 27 g (95%) de produit orangé amorphe.

*Analyse:*

Produit soluble dans l'eau.

Insoluble dans le méthanol et l'acétone.

Calculé: C 42 H 4,56 O 28 N 9,8 Mg 4,26%

Trouvé: C 41,4 H 5,02 O 27,8 N 9,8 Mg 4,02%

Teneur en eau <2%

*Exemple 14*

*N-[(méthyl-2 hydroxy-3 hydroxyméthyl-5 pyridyl-4)-méthylène]γ-imino-propane-sulfonate de magnésium*

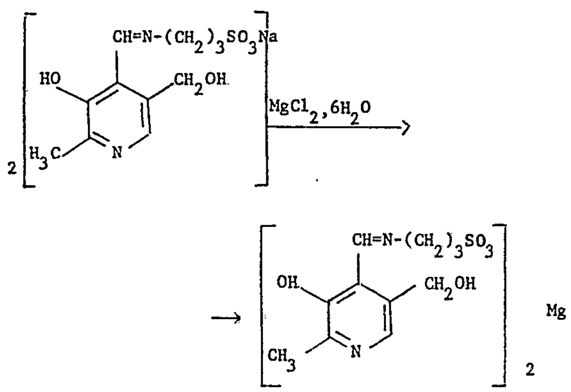


31 g (0,1 mol) du sel de sodium de l'exemple 5 sont mis en suspension dans 180 $cm^3$ de méthanol. On ajoute alors une solution de 10,2 g (0,05 mol) de chlorure de magnésium hydraté dans 20 $cm^3$ de méthanol. Il y a dissolution presque complète et précipitation du chlorure de sodium.

On agite pendant 30 min et on filtre. Le filtrat est concentré à sec et déshydraté sous vide. Le solide est repris avec 100 $cm^3$ de $CH_3OH$ bouillant et essoré à chaud.

On sèche à l'étuve sous vide à 80° C.

On obtient 29 g (97%) de produit orangé amorphe.

*Analyse:*

Produit soluble dans l'eau.

Insoluble dans le méthanol et l'acétone.

Calculé: C 44,1 H 5 N 9,35 O 26,7 Mg 4,06%

Trouvé: C 43,9 H 4,85 N 9,48 O 27 Mg 3,72%

Teneur en eau <2%

On peut, par le même procédé que celui décrit pour les sels de Ca et de Mg, préparer les sels d'autres métaux alcalino-terreux tels que Sr, Ba, etc.

*Etude pharmacologique des composés selon l'invention:*

*Mise en évidence de la propriété vectrice cationique*

L'étude comparée des rapports entre la toxicité par voie orale ($DL_{50}$ par tubage gastrique = B) et par voie parentérale ($DL_{50}$ i.p. = P) des sels alcalins ou alcalino-terreux (rapports B/P) avec celle des sels de référence (chlorures alcalins ou alcalino-terreux correspondants) a permis de mettre en évidence, de façon imprévisible, que le rapport B/P est, pour chaque cation, nettement plus favorable, c'est-à-dire signifcativement plus bas que celui du sel de référence correspondant.

1. *Sels de magnésium:*

L'étude a été effectuée chez la souris Swiss I.O.P.S. par lots de 20 animaux des deux sexes. Le rapport B/P exprimé en millimoles par kilo est de 19,2/9,4 = 2,04 pour le $Cl_2Mg$, sel de référence. Ceux des sels de Mg selon l'invention, c'est-à-dire ceux des exemples 13 et 14, se trouvent considérablement abaissés et de façon hautement significative ($p<0,01$ à l'analyse de variance) par rapport au sel de référence puisque respectivement égaux à 3,3/2,8 = 1,17 et 4,1/3,3 = 1,24. Ils ne sont, par contre, pas significativement différents entre eux.

Ainsi, les sels de Mg selon l'invention se trouvent être presque aussi actifs (selon ce test) par voie orale que par voie parentérale, ce qui est tout à fait exceptionnel pour un sel de Mg.

On a vérifié que cette constatation n'est pas propre à une espèce animale, en effectuant la même observation chez le rat Sprague-Dawley I.O.P.S. Alors que, chez cette espèce, le rapport B/P est, pour le chlorure de Mg, supérieur à 39,3/4,4 = 8,89, il se trouve réduit de façon très hautement significative ($p<0,001$) pour les sels étudiés, puisque égaux, respectivement pour les composés des exemples 13 et 14, à 3,3/2,2 = 1,5 et 4,2/2,4 = 1,66.

Là encore est vérifiée l'exceptionnelle efficacité, selon ce test, des sels de Mg selon l'invention, les rapports pour les deux composés (imino et amino) n'étant pas, entre eux, dans les deux espèces animales, significativement différents.

2. *Sels de calcium:*

Des constatations parallèles ont été effectuées pour les sels de Ca. Alors, par exemple, que chez la souris Swiss I.O.P.S, le rapport B/P est, pour le $Cl_2Ca$, supérieur à 36,5/3,9 = 9,12, il est réduit respectivement pour les sels des exemples 11 et 12 à 7,7/2,6 = 2,96 et à 8,1/2,4 = 3,3, la réduction, là encore, des deux sels n'étant pas, entre eux, significativement différente.

3. *Sels de potassium:*

Des constatations parallèles ont été effectuées pour les sels de potassium. Alors que, par exemple, chez la souris Swiss I.O.P.S. le rapport est, pour le chlorure de potassium (sel de référence), supérieur à 107,3/14,8 = 7,25, il est réduit, pour les composés imino des exemples 3 et 7, respectivement à

7,0/3,9 = 1,79 et 7,0/4,0 = 1,75; pour les composés des exemples 4 et 8, les rapports sont respectivement de 15,7/13,0 = 1,20 et 17,0/14,2 = 1,19. Là encore, il existe une baisse très hautement significative ($p < 0,001$) du rapport B/P attestant l'efficacité des composés. De plus, il faut noter que les composés amino possèdent, comme les composés imino, des propriétés vectrices inattendues. Les rapports des quatre composés des exemples ci-dessus, c'est-à-dire des composés imino et amino, ne sont pas significativement différents entre eux.

4. *Sels de sodium:*

Alors que le rapport est, pour le sel de référence, le chlorure de sodium, supérieur à 205,4/61,2 = 3,36, il se situe, pour les composés imino des exemples 1 et 5, respectivement aux taux de 7,0/3,0 = 2,33 et de 6,7/4,4 = 1,52, et, pour les composés amino des exemples 2 et 6, à des taux non srictement déterminés, mais sans doute supérieurs à 1: respectivement supérieurs à 20,1/20,1 = 1 et supérieurs à 19,2/13 = 1,43. Là encore, les composés imino et amino correspondants possèdent les mêmes propriétés vectrices cationiques.

La haute pénétration cellulaire des composés selon l'invention n'est pas attestée seulement par un rapport B/P favorable, mais cette propriété est traduite également par les résultats des tests d'activité pharmacologique.

A titre d'exemple, l'ensemble des composés ont été examinés, d'une part, par le test de Macallum A.B. et Sivertz C. («Can. Chem. and Process Industries», 1942, *26*, 569) qui ont étudié la potentialisation de l'insuline par les sulfones chez le lapin et, d'autre part, par le test de Fariello G.T. et coll., «Neurology», 1980, *30*, 386), selon lequel ces auteurs ont montré que l'homotaurine antagonise l'action neurotoxique ou convulsivante de l'acide kaïnique administré au rat par voie générale.

On a ainsi constaté, selon le premier test, que les composés selon l'invention, administrés par voie orale, potentialisent l'action hypoglycémiante d'une dose non convulsivante d'insuline intraveineuse, alors que la taurine et l'homotaurine, qui sont actives par voie intraveineuse, ne le sont pas par voie orale.

Selon le second test, alors que la taurine et l'homotaurine administrées *per os* ne provoquent aucune diminution significative des effets neurotoxiques de l'acide kaïnique (symptôme observé: «Wet Dog Shaking»), les composés de l'invention, administrés par la même voie, présentent une activité notable.

Ainsi, d'après ces deux tests, l'un métabolique, l'autre neurologique, les composés selon l'invention se sont révélés actifs de façon significative par voie orale, alors que la taurine, l'homotaurine, la vitamine B6 et les sels cationiques de référence correspondant aux sels de l'invention sont inactifs par cette voie.

Ainsi, l'ensemble des composés selon l'invention, tant imino qu'amino, sels de magnésium, de calcium, de lithium, de potassium et de sodium, présentent tous une exceptionnelle efficacité par voie buccale, variant d'une efficacité presque égale à celle de la voie parentérale dans les meilleurs cas, à une efficacité égale au tiers de celle de la voie parentérale dans les cas les moins favorables. Elle est cependant dans tous les cas, et de façon significative, accrue par rapport aux sels cationiques de référence correspondants, c'est-à-dire aux chlorures correspondants.

*Application thérapeutique:*

Les propriétés vectrices cationiques peuvent être utilisées pour faire pénétrer les cations respectifs, dans tous les cas où existe une déperdition ionique correspondante, rebelle aux thérapeutiques usuelles. Ainsi, les sels de sodium, de potassium, de lithium, de calcium et de magnésium selon l'invention peuvent être utilisés pour pallier les déplétions sodiques, potassiques, lithiques, calciques et magnésiques, rebelles au traitement habituel. Les déplétions magnésiques ne répondant pas à la magnésothérapie sont en particulier fréquentes. On ne dispose jusqu'ici en thérapeutique que de sels de magnésium médiocrement absorbés et pénétrant mal dans la cellule. La baisse du magnésium érythrocytaire utilisé comme témoin d'un déficit intracellulaire se révèle souvent rebelle à la recharge magnésique.

Or, en carençant en magnésium, par un régime ne contenant que 25 ppm de magnésium, des rats Sprague-Dawley I.O.P.S., nous avons pu vérifier, en utilisant diverses modalités de recharge magnésique par voie orale, effectuées avec les deux sels des exemples 13 et 14, l'efficacité de ces sels. On peut vérifier par exemple la réduction de l'aggressivité des rats carencés vis-à-vis des souris (réduction de l'activité muricide) mais surtout, de façon imprévisible, on a pu constater avec diverses posologies (forte: 40 mg de magnésium/kg/d, moyenne: 20 mg de magnésium/kg/d, et faible: 5 mg de magnésium/kg/d) un effet prioritaire de correction de la baisse du magnésium érythrocytaire par rapport à celle du magnésium plasmatique. Cela ne peut s'expliquer que par une pénétration sélective des sels magnésiques selon l'invention à travers la membrane du globule rouge. La correction peut s'observer dès le premier jour; une hypercorrection a même pu être observée au troisième jour (respectivement 79,2 mg pour le composé de l'exemple 13 et 75,9 mg pour le composé de l'exemple 14, les animaux carencés présentant un taux de 40 mg et les animaux témoins un taux de 60 mg).

A l'opposé, aux doses jusqu'ici testées, la correction de la baisse du magnésium plasmatique dans ces carences expérimentales subaiguës ne s'est jamais produite avant le troisième jour d'administration des composés. Ces délais demeurent toutefois toujours plus courts que ceux observés avec le sel de magnésium de référence (chlorure de magnésium).

*Autres propriétés:*

Les propriétés générales vectrices cationiques des composés selon l'invention n'excluent en aucune façon d'autres utilisations thérapeutiques particulières de ces composés, certaines propriétés pouvant être très différentes selon la stucture de l'anion, notamment saturé ou non, et la nature du cation.

On peut, par exemple, noter que, sur l'ensemble des composés selon l'invention, seul l'un d'eux, c'est-à-dire le composé de l'exemple 8 testé selon le test de H. Frances et P. Simon («Prog. Neuropsychopharmacol.», 1977, *1*, 227 à 230), présente une activité analogue à celle de la taurine, mais considérablement potentialisée, c'est-à-dire 17 fois plus active.

*Tableau: résultats du test de Frances et Simon*

| Composé | $DE_{50}$ roulements (mmol/kg) | $DE_{50}$ tremblements (mmol/kg) |
|---|---|---|
| Taurine | 4,67 | 4,34 |
| Composé de l'exemple 8 | 0,27 | 0,27 |

*Forme galénique:*

Si la solubilité dans l'eau des composés selon l'invention permet d'en envisager l'administration parentérale en ampoules destinées aux voies sous-cutanée, intramusculaire et intraveineuse, la voie d'administration préférée sera la voie orale et on pourra utiliser toute forme galénique, c'est-à-dire: comprimé, gélule, granulé, soluté, sirop. Ne sont pas exclus les suppositoires et les formes d'administration locale de ces produits de haute pénétration cellulaire telles que collyres, gouttes nasales ou auriculaires, pommades.

A titre d'exemple de préparation galénique, on a préparé selon les procédés galéniques classiques:

— des gélules dosées à 250 mg du composé de l'exemple 13,

— des ampoules buvables de 5 ml, dosées à 200 mg, du composé de l'exemple 12,

— un soluté à 10% dans l'eau du composé de l'exemple 8.

La dose unitaire des diverses préparations peut varier de 10 à 1000 mg.

*Exemple d'utilisation thérapeutique:*

M^me^ Ser... Claude est atteinte de tétanie latente par déficit magnésique; elle est traitée depuis deux ans par diverses modalités de magnésothérapie: 3 g/d de lactate de magnésium ou trois ampoules buvables par jour de pyroglutamate de magnésium titrant 127 mg de $Mg^{++}$ par ampoule, sans parvenir à corriger un magnésium érythrocytaire variant de 43 à 46 mg/l. La prescription de trois gélules du composé de l'exemple 13 amène une régression des signes cliniques (céphalée, asthénie, crampes...) et la correction, le premier mois, à 48 mg/l et, le second mois, à 53 mg/l du magnésium érythrocytaire. Ultérieurement, on doit cependant observer que chaque interruption d'administration du produit est suivie, cliniquement et biologiquement, au contrôle mensuel suivant, d'une rechute (reprise de la symptomatologie et baisse du magnésium érythrocytaire aux environs de 45 mg/l). En conclusion, un apport quotidien modéré de 32 mg d'ion magnésium sous la forme de 750 mg du composé de l'exemple 13 s'est révélé plus actif pour contrôler un déficit magnésique rebelle que des prescriptions de sels de magnésium classiques à des doses dix fois plus grandes.

**Revendications** pour les Etats contractants BE, CH, DE, FR, GB, IT, LI, LU, NL, SE

1. Sels d'amino-(et imino-)acides α, ω-sulfoniques N-substitués, représentés par l'une ou l'autre des formules (I) et (II) suivantes:

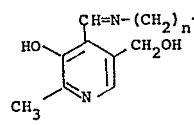 (I) et 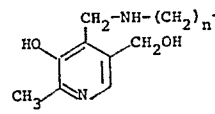 (II)

dans lesquelles n est égal à 2 ou 3 et M est un métal alcalin ou alcalino-terreux.

2. Sels selon la revendication 1, caractérisés en ce qu'ils répondent à la formule (I), dans laquelle n est égal à 2 et M représente un métal choisi parmi les suivants: Na, K, Li, Ca, Mg.

3. Sels selon la revendication 1, caractérisés en ce qu'ils répondent à la formule (I), dans laquelle n est égal à 3 et M représente un métal choisi parmi les suivants: Na, K, Li, Ca, Mg.

4. Sels selon la revendication 1, caractérisés en ce qu'ils répondent à la formule (II), dans laquelle n est égal à 2 ou 3 et M représente un métal choisi parmi les suivants: Na, K, Li.

5. Compositions pharmaceutiques administrables par voie orale, locale ou parentérale, caractérisées en ce qu'elles contiennent comme principe actif au moins l'un des composés selon l'une des revendications 1 à 4.

6. Compositions pharmaceutiques selon la revendication 5, caractérisées en ce qu'elles contiennent le principe actif à la dose unitaire de 10 à 1000 mg, avec un excipient pharmaceutique acceptable pour la voie orale et sont présentées sous forme de gélules, comprimés, solutés ou ampoules buvables.

7. Compositions selon la revendication 5, caractérisées en ce qu'elles sont présentées sous forme de suppositoires, ou de collyres, gouttes ou pommades nasales ou auriculaires.

8. Procédé pour l'obtention des sels selon l'une des revendications 1 à 4, caractérisé en ce qu'il consiste:

1) à faire réagir le pyridoxal base avec le sel d'un métal alcalin de l'acide α, ω-amino-sulfonique de formule $NH_2-(CH_2)_n-SO_3H$, dans laquelle n est égal à 2 ou 3, en présence d'une base en milieu

alcoolique pour former le composé imino de formule (I) dans laquelle M est un métal alcalin;

2) éventuellement à transformer le sel alcalin en le sel alcalino-terreux du composé obtenu à l'étape 1, par échange entre ledit sel alcalin et un sel alcalino-terreux, par exemple un chlorure;

3) éventuellement à réduire le dérivé imino obtenu selon l'une des étapes 1 ou 2 en le dérivé amino correspondant.

9. Procédé selon la revendication 8, caractérisé en ce que l'on utilise le sel de sodium, de potassium ou de lithium de l'acide α, ω-amino-sulfonique de formule $NH_2-(CH_2)_n-SO_3H$ dans laquelle n est égal à 2 ou 3.

10. Procédé selon la revendication 8, caractérisé en ce qu'il comprend les étapes 1 et 3 et en ce qu'on utilise le sel de sodium, de potassium ou de lithium de l'acide α, ω-amino-sulfonique de formule $NH_2-(CH_2)_n-SO_3H$ dans laquelle n est égal à 2 ou 3.

11. Procédé selon la revendication 8, caractérisé en ce qu'il comprend les étapes 1 et 2 et en ce qu'on utilise dans l'étape 1 le sel de sodium de l'acide α, ω-amino-sulfonique de formule $NH_2-(CH_2)_n-SO_3H$ dans laquelle n est égal à 2 ou 3 et en ce que l'échange dans l'étape 2 est réalisé avec le chlorure de magnésium ou de calcium.

12. Procédé selon la revendication 8, caractérisé en ce qu'il comprend les étapes 1 à 3 et en ce qu'on utilise dans l'étape 1 le sel de sodium de l'acide α, ω-amino-sulfonique de formule $NH_2-(CH_2)_n-SO_3H$ dans laquelle n est égal à 2 ou 3 et en ce que l'échange dans l'étape 2 est réalisé avec le chlorure de magnésium ou de calcium.

**Revendications** pour l'Etat contractant: AT

1. Procédé pour l'obtention de sels d'amino- (et imino-)acides α, ω-sulfoniques N-substitués, représentés par l'une ou l'autre des formules (I) et (II) suivantes:

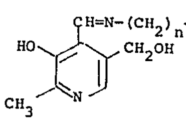
(I) et
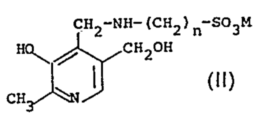
(II)

dans lesquelles n est égal à 2 ou 3 et M est un métal alcalin ou alcalino-terreux, caractérisé en ce qu'il consiste:

1) à faire réagir le pyridoxal base avec le sel d'un métal alcalin de l'acide α, ω-amino-sulfonique de formule $NH_2-(CH_2)_n-SO_3H$ dans laquelle n est égal à 2 ou 3, en présence d'une base en milieu alcoolique pour former le composé imino de formule (I) dans laquelle M est un métal alcalin;

2) éventuellement à transformer le sel alcalin en le sel alcalino-terreux du composé obtenu à l'étape 1, par échange entre ledit sel alcalin et un sel alcalino-terreux, par exemple un chlorure;

3) éventuellement à réduire le dérivé imino obtenu selon l'une des étapes 1 ou 2 en le dérivé amino correspondant.

2. Procédé selon la revendication 1, caractérisé en ce que l'on utilise le sel de sodium, de potassium ou de lithium de l'acide α, ω-amino-sulfonique de formule $NH_2-(CH_2)_n-SO_3H$ dans laquelle n est égal à 2 ou 3.

3. Procédé selon la revendication 1, caractérisé en ce qu'il comprend les étapes 1 et 3 et en ce qu'on utilise le sel de sodium, de potassium ou de lithium de l'acide α, ω-amino-sulfonique de formule $NH_2-(CH_2)_n-SO_3H$ dans laquelle n est égal à 2 ou 3.

4. Procédé selon la revendication 1, caractérisé en ce qu'il comprend les étapes 1 et 2 et en ce qu'on utilise dans l'étape 1 le sel de sodium de l'acide α, ω-amino-sulfonique de formule $NH_2-(CH_2)_n-SO_3H$ dans laquelle n est égal à 2 ou 3 et en ce que l'échange dans l'étape 2 est réalisé avec le chlorure de magnésium ou de calcium.

5. Procédé selon la revendication 1, caractérisé en ce qu'il comprend les étapes 1 à 3 et en ce qu'on utilise dans l'étape 1 le sel de sodium de l'acide α, ω-amino-sulfonique de formule $NH_2-(CH_2)_n-SO_3H$ dans laquelle n est égal à 2 ou 3 et en ce que l'échange dans l'étape 2 est réalisé avec le chlorure de magnésium ou de calcium.

6. Utilisation des sels d'amino- (et imino-) acides α, ω-sulfoniques N-substitués représentés par l'une ou l'autre des formules (I) et (II) suivantes:

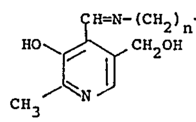
(I) et
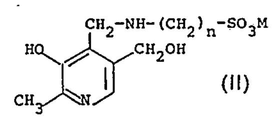
(II)

dans lesquelles n est égal à 2 ou 3 et M est un métal alcalin ou alcalino-terreux, pour la préparation de compositions pharmaceutiques administrables par voie orale, locale ou parentérale, contenant à titre de principe actif au moins l'un desdits composés définis ci-dessus.

7. Utilisation selon la revendication 6 pour la préparation de compositions pharmaceutiques contenant le principe actif à la dose unitaire de 10 à 1000 mg avec un excipient pharmaceutiquement acceptable pour la voie orale, lesdites compositions étant présentées sous forme de gélules, comprimés, solutés ou ampoules buvables.

8. Utilisation selon la revendication 6 pour la préparation de compositions pharmaceutiques présentées sous forme de suppositoires, de collyres, gouttes ou pommades nasales ou auriculaires.

**Claims** for the Contracting States BE, CH, DE, FR, GB, IT, LI, LU, NL, SE

1. N-substituted α, ω-amino-(and imino-) sulfonic acids, represented by either of the following formulae (I) and (II):

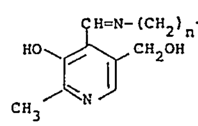
(I) and
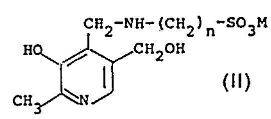
(II)

in which n is equal to 2 or 3 and M is an alkali metal or alkaline earth metal.

2. Salts according to Claim 1, characterized in that they respond to formula (I), in which n is

equal to 2 and M is a metal selected from Na, K, Li, Ca, Mg.

3. Salts according to Claim 1, characterized in that they respond to formula (I), in which n is equal to 3 and M is a metal selected from Na, K, Li, Ca, Mg.

4. Salts according to Claim 1, characterized in that they respond to formula (II), in which n is equal to 2 or 3 and M is a metal selected from Na, K, Li.

5. Pharmaceutical compositions which can be administered by oral, local or parenteral route containing as active principle at least one of the compounds according to any one of Claims 1 to 4.

6. Pharmaceutical compositions according to Claim 5, characterized in that they contain the active principle at the unit dose of 10 to 1,000 mg with a pharmaceutically acceptable excipient for oral administration, said composition being in the form of capsules, tablets, solutions, drinkable ampullae.

7. Compositions according to Claim 5, characterized in that they are in the form of suppositories, collyria, nasal or ear drops or ointments.

8. Process for obtaining salts according to any one of Claims 1 to 4, characterized in that it consists:

1) in reacting the pyridoxal base with the salt of an alkali metal of α, ω-aminosulfonic acid of formula $NH_2-(CH_2)_n-SO_3H$ in which n is equal to 2 or 3, in the presence of a base in alcoholic medium to form the imino compound of formula (I), in which M is an alkali metal;

2) optionally converting the alkali metal into the alkaline earth salt of the compound obtained in step 1), by exchange between said alkaline salt and an alkaline earth salt, for example a chloride;

3) optionally reducing the imino derivative obtained according to step 1) or 2) into the corresponding amino derivative.

9. Process according to Claim 8, characterized in that the sodium, potassium or lithium salt of the α, ω-aminosulfonic acid of formula $NH_2-(CH_2)_n-SO_3H$, in which n is equal to 2 or 3, is used.

10. Process according to Claim 8, characterized in that it comprises steps 1) and 3) and in that the sodium, potassium or lithium salt of the α, ω-aminosulfonic acid of formula $NH_2-(CH_2)_n-SO_3H$, in which n is equal to 2 or 3, is used.

11. Process according to claim 8, characterized in that it comprises steps 1) and 2) and in that the sodium salt of α, ω-aminosulfonic acid of formula $NH_2-(CH_2)_n-SO_3H$, in which n is equal to 2 or 3, is used in step 1), and in that the exchange in step 2) is carried out with the magnesium or calcium chloride.

12. Process according to Claim 8, characterized in that it comprises steps 1) to 3) and in that the sodium salt of α, ω-aminosulfonic acid of formula $NH_2-(CH_2)_n-SO_3H$, in which n is equal to 2 or 3, is used in step 1), and in that the exchange in step 2) is carried out with the magnesium or calcium chloride.

**Claims** for the Contracting State: AT

1. Process for obtaining N-substituted α, ω-amino-(and imino-) sulfonic acids, represented by either of the following formulae (I) and (II):

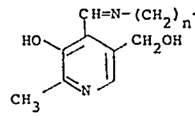
(I) and
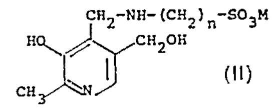
(II)

in which n is equal to 2 or 3 and M is an alkali metal or alkaline earth metal, characterized in that it consists:

1) in reacting the pyridoxal base with the salt of an alkali metal of α, ω-aminosulfonic acid of formula $NH_2-(CH_2)_n-SO_3H$, in which n is equal to 2 or 3, in the presence of a base in alcoholic medium to form the imino compound of formula (I), in which M is an alkali metal;

2) optionally converting the alkali metal into the alkaline earth salt of the compound obtained in step 1), by exchange between said alkaline salt and an alkaline earth salt, for example a chloride;

3) optionally reducing the imino derivative obtained according to step 1) or 2) into the corresponding amino derivative.

2. Process according to Claim 1, characterized in that the sodium, potassium or lithium salt of the α, ω-aminosulfonic acid of formula $NH_2-(CH_2)_n-SO_3H$, in which n is equal to 2 or 3, is used.

3. Process according to Claim 1, characterized in that it comprises steps 1) and 3) and in that the sodium, potassium or lithium salt of the α, ω-aminosulfonic acid of formula $NH_2-(CH_2)_n-SO_3H$, in which n is equal to 2 or 3, is used.

4. Process according to Claim 1, characterized in that it comprises steps 1) and 2), and in that the sodium salt of α, ω-aminosulfonic acid of formula $NH_2-(CH_2)_n-SO_3H$, in which n is equal to 2 or 3, is used in step 1), and in that the exchange in step 2) is carried out with the magnesium or calcium chloride.

5. Process according to Claim 1, characterized in that it comprises steps 1) to 3), and in that the sodium salt of α, ω-aminosulfonic acid of formula $NH_2-(CH_2)_n-SO_3H$, in which n is equal to 2 or 3, is used in step 1), and in that the exchange in step 2) is carried out with the magnesium or calcium chloride.

6. Use of N-substituted α, ω-amino-(and imino-) sulfonic acids represented by either of the following formulae (I) and (II):

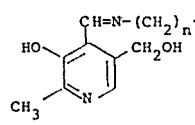
(I) and
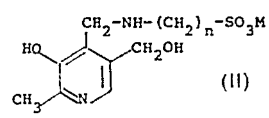
(II)

in which n is equal to 2 or 3 and M is an alkali metal or alkaline earth metal, for the preparation of pharmaceutical compositions which can be administered by oral, local or parenteral route containing as active principle at least one of said compounds defined above.

7. Use according to Claim 6, for the preparation of pharmaceutical compositions containing the active principle at the unit dose of 10 to 1,000 mg with a pharmaceutically acceptable excipient for oral administration, said composition being in the form of capsules, tablets, solutions, drinkable ampullae.

8. Use according to Claim 6, for the preparation of pharmaceutical compositions in the form of suppositories, collyria, nasal or ear drops or ointments.

**Patentansprüche** für die Vertragsstaaten BE, CH, DE, FR, GB, IT, LI, LU, NL, SE

1. Salze von N-substituierten α-Amino-(und Imino-)-ω-sulfonsäuren der einen oder der anderen der folgenden Formel (I) bzw. (II):

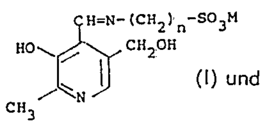
(I) und
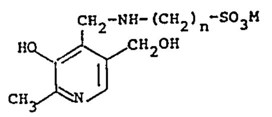

worin n gleich 2 oder 3 ist und M für ein Alkali- oder Erdalkalimetall steht.

2. Salze nach Anspruch 1, dadurch gekennzeichnet, dass sie der Formel (I) entsprechen, worin n gleich 2 ist und M für ein Metall, ausgewählt aus Na, K, Li, Ca, Mg, steht.

3. Salze nach Anspruch 1, dadurch gekennzeichnet, dass sie der Formel (I) entsprechen, worin n gleich 3 ist und M für ein Metall, ausgewählt aus Na, K, Li, Ca, Mg, steht.

4. Salze nach Anspruch 1, dadurch gekennzeichnet, dass sie der Formel (II) entsprechen, worin n gleich 2 oder 3 ist und M für ein Metall, ausgewählt aus Na, K, Li, steht.

5. Auf oralem, lokalem oder parenteralem Weg verabreichbare pharmazeutische Zusammensetzungen, dadurch gekennzeichnet, dass sie als Wirkstoff mindestens eine der Verbindungen nach einem der Ansprüche 1 bis 4 enthalten.

6. Pharmazeutische Zusammensetzungen nach Anspruch 5, dadurch gekennzeichnet, dass sie den Wirkstoff in der Einheitsdosis von 10 bis 1000 mg mit einem pharmazeutisch akzeptablen Exzipienten für oralen Weg enthalten und in Form von Kapseln, Tabletten, gelösten Stoffen oder Trinkampullen vorliegen.

7. Zusammensetzungen nach Anspruch 5, dadurch gekennzeichnet, dass sie in Form von Suppositorien oder Nasen- oder Ohrenwässern, -tropfen oder -salben vorliegen.

8. Verfahren zur Herstellung der Salze nach einem der Ansprüche 1 bis 4, dadurch gekennzeichnet, dass

1) die Pyridoxalbase mit dem Alkalimetallsalz der α-Amino-ω-sulfonsäure der Formel $NH_2-(CH_2)_n-SO_3H$, worin n gleich 2 oder 3 ist, in Gegenwart einer Base in alkoholischem Milieu zur Bildung der Iminoverbindung der Formel (I), worin M ein Alkalimetall ist, zur Umsetzung gebracht wird;

2) gegebenenfalls das Alkalisalz der in Stufe 1 erhaltenen Verbindung durch Austausch zwischen dem Alkalisalz und einem Erdalkalisalz, beispielsweise einem Chlorid, in das Erdalkalisalz umgewandelt wird;

3) gegebenenfalls das nach einer der Stufen 1 oder 2 erhaltene Iminoderivat zu dem entsprechenden Aminoderivat reduziert wird.

9. Verfahren nach Anspruch 8, dadurch gekennzeichnet, dass das Natrium-, Kalium- oder Lithiumsalz der α-Amino-ω-sulfonsäure der Formel $NH_2-(CH_2)_n-SO_3H$, worin n gleich 2 oder 3 ist, eingesetzt wird.

10. Verfahren nach Anspruch 8, dadurch gekennzeichnet, dass es die Stufen 1 und 3 umfasst und das Natrium-, Kalium- oder Lithiumsalz der α-Amino-ω-sulfonsäure der Formel $NH_2-(CH_2)_n-SO_3H$, worin n gleich 2 oder 3 ist, eingesetzt wird.

11. Verfahren nach Anspruch 8, dadurch gekennzeichnet, dass es die Stufen 1 und 2 umfasst und in der Stufe 1 das Natriumsalz der α-Amino-ω-sulfonsäure der Formel $NH_2-(CH_2)_n-SO_3H$, worin n gleich 2 oder 3 ist, eingesetzt wird, und dass der Austausch in Stufe 2 mittels Magnesium- oder Calciumchlorid durchgeführt wird.

12. Verfahren nach Anspruch 8, dadurch gekennzeichnet, dass es die Stufen 1 bis 3 umfasst und in der Stufe 1 das Natriumsalz der α-Amino-ω-sulfonsäure der Formel $NH_2-(CH_2)_n-SO_3H$, worin n gleich 2 oder 3 ist, eingesetzt wird, und dass der Austausch in Stufe 2 mit Magnesium- oder Calciumchlorid durchgeführt wird.

**Patentansprüche** für den Vertragsstaat: AT

1. Verfahren zur Herstellung der Salze von N-substituierten α-Amino-(und Imino-)-ω-sulfonsäuren der einen oder der anderen der folgenden Formel (I) bzw. (II):

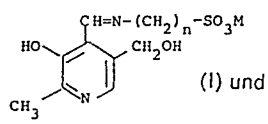
(I) und
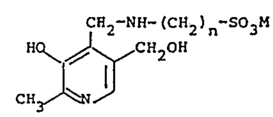

worin n gleich 2 oder 3 ist und M für ein Alkali- oder Erdalkalimetall steht, dadurch gekennzeichnet, dass es darin besteht, dass

1) die Pyridoxalbase mit dem Alkalimetallsalz der α-Amino-ω-sulfonsäure der Formel $NH_2-(CH_2)_n-SO_3H$, worin n gleich 2 oder 3 ist, in Gegenwart einer Base in alkoholischem Milieu zur Bildung der Iminoverbindung der Formel (I), worin M ein Alkalimetall ist, zur Umsetzung gebracht wird;

2) gegebenenfalls das Alkalisalz der in Stufe 1 erhaltenen Verbindung durch Austausch zwischen dem Alkalisalz und einem Erdalkalisalz, beispielsweise einem Chlorid, in das Erdalkalisalz umgewandelt wird;

3) gegebenenfalls das nach einer der Stufen 1 oder 2 erhaltene Iminoderivat zu dem entsprechenden Aminoderivat reduziert wird.

2. Verfahren nach Anspruch 1, dadurch gekennzeichnet, dass das Natrium-, Kalium- oder Lithiumsalz der α-Amino-ω-sulfonsäure der Formel $NH_2-(CH_2)_n-SO_3H$, worin n gleich 2 oder 3 ist, eingesetzt wird.

3. Verfahren nach Anspruch 1, dadurch gekennzeichnet, dass es die Stufen 1 und 3 umfasst und das Natrium-, Kalium- oder Lithiumsalz der α-Amino-ω-sulfonsäure der Formel $NH_2-(CH_2)_n-SO_3H$, worin n gleich 2 oder 3 ist, eingesetzt wird.

4. Verfahren nach Anspruch 1, dadurch gekennzeichnet, dass es die Stufen 1 und 2 umfasst und in der Stufe 1 das Natriumsalz der α-Amino-ω-sulfonsäure der Formel $NH_2-(CH_2)_n-SO_3H$, worin n gleich 2 oder 3 ist, eingesetzt wird, und dass der Austausch in Stufe 2 mittels Magnesium- oder Calciumchlorid durchgeführt wird.

5. Verfahren nach Anspruch 1, dadurch gekennzeichnet, dass es die Stufen 1 bis 3 umfasst und in der Stufe 1 das Natriumsalz der α-Amino-ω-sulfonsäure der Formel $NH_2-(CH_2)_n-SO_3H$, worin n gleich 2 oder 3 ist, eingesetzt wird, und dass der Austausch in Stufe 2 mit Magnesium- oder Calciumchlorid durchgeführt wird.

6. Verwendung der Salze von N-substituierten α-Amino-(und Imino-)-ω-sulfonsäuren der einen oder der anderen der folgenden Formel (I) bzw. (II):

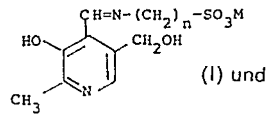
(I) und
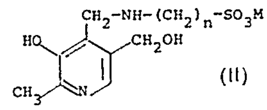
(II)

worin n gleich 2 oder 3 ist und M für ein Alkali- oder Erdalkalimetall steht, zur Herstellung von auf oralem, lokalem oder parenteralem Weg verabreichbaren pharmazeutischen Zusammensetzungen, die als Wirkstoff mindestens eine der oben definierten Verbindungen enthalten.

7. Verwendung nach Anspruch 6 zur Herstellung von pharmazeutischen Zusammensetzungen, die den Wirkstoff in der Einheitsdosis von 10 bis 1000 mg mit einem pharmazeutisch akzeptablen Exzipienten für oralen Weg enthalten, wobei die Zusammensetzungen in Form von Kapseln, Tabletten, gelösten Stoffen oder Trinkampullen vorliegen.

8. Verwendung nach Anspruch 6 zur Herstellung von pharmazeutischen Zusammensetzungen, die in Form von Suppositorien, Nasen- oder Ohrenwässern, -tropfen oder -salben vorliegen.